# EUROPÄISCHE PATENTSCHRIFT

(11) **EP 2 052 748 B1**
(45) Veröffentlichungstag und Bekanntmachung des Hinweises auf die Patenterteilung: **04.01.2017**
(21) Anmeldenummer: 08016666.3
(22) Anmeldetag: 23.09.2008
(51) Int. Cl.: A61L 24/06, A61L 24/04

(54) **Pastenförmiger Polymethylmethacrylat-Knochenzement**
Polymethyl methacrylate bone cement in paste form
Ciment osseux sous forme de pâte en polyméthylméthacrylate

(30) Priorität: 22.10.2007 DE 102007050762
(43) Veröffentlichungstag der Anmeldung: 29.04.2009
(73) Patentinhaber: Heraeus Medical GmbH, 61273 Wehrheim (DE)
(72) Erfinder: Vogt, Sebastian, Dr., 99092 Erfurt (DE); Büchner, Hubert, Dr., 64354 Reinheim (DE)
(74) Vertreter: Heraeus IP

(56) Entgegenhaltungen:
- DE-A1- 19 928 238
- US-A- 4 369 262
- US-A- 4 396 476
- US-A- 5 797 873

## Beschreibung

Gegenstand der Erfindung ist ein pastenförmiger Polymethylmethacrylat-Knochenzement (PMMA-Knochenzement).

PMMA-Knochenzemente sind seit Jahrzehnten bekannt und gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Der Grundaufbau der PMMA-Knochenzemente ist seitdem prinzipiell gleich geblieben. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente besteht aus einem oder mehreren Polymeren, die auf Basis von Methylmethacrylat und Comonomeren, wie Styren, Methylacrylat oder ähnlichen Monomeren durch Polymerisation, vorzugsweise Suspensionspolymerisation, hergestellt sind, einem Röntgenopaker und dem Initiator Dibenzoylperoxid. Beim Vermischen der Pulverkomponente mit der Monomerkomponente entsteht durch Quellung der Polymere der Pulverkomponente im Methylmethacrylat ein plastisch verformbarer Teig. Gleichzeitig reagiert der Aktivator N,N-dimethyl-p-toluidin mit dem Dibenzoylperoxid, dass unter Bildung von Radikalen zerfällt. Die gebildeten Radikale initiieren die radikalische Polymerisation des Methylmethacrylates. Mit fortschreitender Polymerisation des Methylmethacrylates erhöht sich die Viskosität des Zementteigs, bis der Zementteig erstarrt und damit ausgehärtet ist.

Die grundlegenden mechanischen Anforderungen an PMMA-Knochenzemente, wie 4-Punkt-Biegefestigkeit, Biegemodul und Druckfestigkeit, sind in der ISO 5833 beschrieben. Für den Anwender der PMMA-Knochenzemente ist die Eigenschaft der Klebfreiheit des Knochenzementes von wesentlicher Bedeutung. Der Begriff Klebfreiheit ist in der ISO5833 definiert. Die Klebfreiheit zeigt bei konventionellen PMMA-Knochenzementen an, dass der Zement nach dem Vermischen der Komponenten durch Quellung der im Zementpulver enthaltenen Polymere im Monomer die Verarbeitungsphase erreicht hat. Grundsätzlich muss ein PMMA-Knochenzement klebfrei sein, damit der Anwender den Zement formen und applizieren kann. Der PMMA-Knochen-zement darf nicht an den Handschuhen und an Applikationshilfen, wie Mischsystemen, Tiegeln oder Spateln kleben.

Vorteilhaft sind gefärbte PMMA-Knochenzemente, damit der PMMA-Knochenzement problemlos vom Knochengewebe visuell differenziert werden kann. Seit Jahrzehnten werden deshalb die PMMA-Knochenzemente der Firma Heraeus Kulzer GmbH/Heraeus Medical GmbH mit gekupfertem Chlorophyll (Chlorophyllin), das als Lebensmittelfarbstoff E141 bekannt ist, gefärbt. In DE102005033210 wird ein farbiger PMMA-Knochenzement vorgeschlagen, der dadurch charakterisiert ist, dass A in der Monomerflüssigkeit ein in Methacrylsäuremethylester nicht oder gering löslicher (s) Farbstoff/Farbstoffgemisch und ein synthetisch hergestellter, proteinfreier, hydrophober, niedermolekularer oder oligomerer Lösungsvermittler für den Farbstoff oder das Farbstoffgemisch gelöst sind, B dass die Monomerflüssigkeit bei Raumtemperatur sichtklar ist, und C dass ein in Methacrylsäuremethylester nicht oder gering löslicher(s) Farbstoff/Farbstoffgemisch und ein synthetisch, hergestellter, proteinfreier, hydrophober, niedermolekularer oder oligomerer Lösungsvermittler, homogen im Polymethacrylat oder Polymethylmethacrylat gelöst ist. Das bedeutet, durch einen synthetischen Lösungsvermittler können an sich in Methylmethacrylat nicht lösliche Farbstoffe/Farbstoffe zur homogenen Färbung der Monomerkomponente und der Pulverkomponente von PMMA-Knochenzemente verwendet werden. Dagegen wird bei DE102005032110 eine andere Variante zur Färbung von PMMA-Knochenzementen offengelegt. Es wird ein gefärbter PMMA-Knochenzement beschrieben, der dadurch charakterisiert ist, dass mindestens die Oberfläche der Polymerpartikel der Pulverkomponente mit einem Gemisch aus einem oder mehreren Farbstoffen und einem hydrophoben, niedermolekularen oder oligomere, organischen Haftvermittler teilweise oder vollständig beschichtet ist, wobei eine solche Menge an Haftvermittler vorhanden ist, dass die Polymerpartikel visuell erkennbar nicht angequollen sind.

Der wesentliche Nachteil der bisherigen PMMA-Knochenzemente für den medizinischen Anwender besteht darin, dass der Anwender die flüssige Monomerkomponente mit der Pulverkomponente unmittelbar vor der Zementapplikation in einem Mischsystem oder in Tiegeln vermischen muss. Dabei können leicht Mischungsfehler auftreten, welche die Zementqualität negativ beeinflussen können. Das Vermischen der Komponenten muss zügig erfolgen. Dabei kommt es darauf an, dass das gesamte Zementpulver ohne Klumpenbildung mit der Monomerkomponente vermischt wird und das beim Vermischungsvorgang der Eintrag von Luftblasen vermieden wird. Bei der Verwendung von Vakuummischsystemen wird im Gegensatz zur Handanmischung die Bildung von Luftblasen im Zementteig weitgehend verhindert, jedoch ist für diese Systeme eine zusätzliche Vakuumpumpe erforderlich. Beispiele für Mischsysteme sind in den Schriften US 5,797,873, US 4,015,945, EP 0 674 888 A1 und JP 2003181270 offengelegt. Vakuummisch-systeme und Vakuumpumpen sind relativ kostenintensiv. Nach der Vermischung der Monomerkomponente mit der Pulverkomponente muss je nach Zementtyp eine mehr oder weniger lange Zeit gewartet werden, bis der Zementteig klebfrei ist und appliziert werden kann. Auf Grund der vielen Fehlermöglichkeiten beim Anmischen von konventionellen PMMA-Knochenzementen wird entsprechend geschultes Personal benötigt. Die Schulungen sind mit einem nicht geringen Kostenaufwand verbunden. Weiterhin kommt es bei der Vermischung der flüssigen Monomerkomponente mit der Pulverkomponente zu einer Belastung der Anwender durch Monomerdämpfe und durch Freisetzung von pulverförmigen Zementpartikeln.

Ein wesentlicher Nachteil der konventionellen PMMA-Knochenzemente für den Zementproduzenten besteht darin, dass sowohl die Pulverkomponente als auch Monomerkomponente jeweils doppelt steril verpackt hergestellt werden müssen. Das bedeutet, es sind mindestens vier sterile Packmittel für eine Knochenzementpackung notwendig.

Die Aufgabe der Erfindung besteht deshalb darin, einen PMMA-Knochenzement zu entwickeln, der die dargestellten Nachteile der bekannten PMMA-Knochenzemente lindert oder beseitigt. Der zu entwickelnde PMMA-Knochenzement soll insbesondere in einer Form für den Anwender bereitgestellt werden, dass ein umständliches, mit vielen Fehlermöglichkeiten behaftetes Vermischen von Zementkomponenten vermieden wird. Der Knochenzement soll möglichst einfach applizierbar sein. Der Zement soll so bereitgestellt werden, dass eine Wartephase bis zur Erreichung der Klebfreiheit nicht notwendig ist. Der Zementteig muss eine solche Viskosität und Kohäsion aufweisen, dass er dem Blutungsdruck bis zur Aushärtung standhält. Weiterhin soll die Belastung der Anwender durch Monomerdämpfe weitgehend vermieden werden.

Die Erfindung basiert auf der grundsätzlichen Idee, in einem Methacrylatmonomer ein darin lösliches Polymer zu lösen und darin ein partikuläres, nicht im Methacrylatmonomer lösliches Polymer zu suspendieren. Dadurch gelingt es, eine teigartige Masse herzustellen, die durch das gelöste Polymer einen hohen inneren Zusammenhalt zeigt und die durch das partikuläre, unlösliche Polymer eine so hohe Viskosität hat, dass der Teig kurzzeitig dem Blutungsdruck standhalten kann. Durch radikalische Polymerisation des Methacrylatmonomeren kann der Teig ausgehärtet werden. Überraschend ist dabei, dass ein Gewichtsverhältnis von Methacrylat zu löslichem Polymer zu unlöslichem Polymer gefunden wurde, bei dem der Teig klebfrei ist. Wesentlich ist weiterhin, dass bei diesem Gewichtsverhältnis der Komponenten der Teig in Wasser unlöslich ist.

Die Aufgabe der Erfindung wird deshalb durch einem zweikomponentigen pastenförmigen PMMA-Knochenzement gelöst, der dadurch charakterisiert ist, dass eine pastenförmige Komponente A, die aus einem oder mehreren destillierbaren, radikalisch polymerisierbaren Methacrylatmonomeren, mindestens einem im Methacrylatmonomer/Methacrylatmonomeren löslichen Polymethylmethacrylatcopolymer, mindestens einem im Methacrylatmonomer/Methacrylatmonomeren nicht löslichen partikulären vernetzten Polymethylmethacrylat oder vernetzem Polymethylmethacrylat-co-methylacrylat mit einer Partikelgröße kleiner 500 µm und mindestens einem radikalischen Initiator aufgebaut ist und einer pastenförmigen Komponente B, die aus einem oder mehreren destillierbaren, radikalisch polymerisierbaren Methacrylatmonomeren, mindestens einem im Methacrylatmonomer/Methacrylatmonomeren löslichen Polymethylmethacrylat-copolymer, mindestens einem im Methacrylatmonomer/Methacrylatmonomeren nicht löslichen partikulären vernetzten Polymethylmethacrylat oder vernetzem Polymethylmethacrylat-co-methylacrylat mit einer Partikelgröße kleiner 500 µm und mindestens einem Akzelerator aufgebaut ist, vorhanden ist und dass bei der Vermischung der pastenförmigen Komponenten A und B eine klebfreie Paste entsteht, die selbständig aushärtet.

Die Methacrylatmonomere müssen destillierbar sein, damit cytotoxische Verunreinigungen, die bei der Monomersynthese anfallen oder als nicht umgesetzte Edukte im Monomer verbleiben, sicher vom Monomer abgetrennt werden können. Insbesondere Reste von Methacrylsäure und Methacrylsäurechlorid haben eine cytotoxische Wirkung.

Die pastenförmigen Komponenten A und B können durch biokompatible Farbstoffe gefärbt sein, wobei die Färbung nur einer Komponente besonders vorteilhaft ist, damit die Vermischung der beiden Komponenten visuell verfolgt werden kann.

Die Vermischung der pastenförmigen Komponenten A und B kann durch einfaches Verkneten oder Vermischen erfolgen. Es ist möglich, die pastenförmigen Komponenten A und B getrennt in Doppelkartuschen zu lagern und unmittelbar vor der Anwendung mit einem auf die Doppelkartusche aufgesetzten statischen Mischer zu vermischen.

Der besondere Vorteil des erfindungsgemäßen pastenförmigen PMMA-Knochenzementes besteht darin, dass keine komplizierte Mischprozedur durchgeführt werden muss und dass keine Wartephase bis zur Zementapplikation nach dem Vermischen erforderlich ist. Der Zement kann unmittelbar nach dem Vermischen appliziert werden.

Es ist zweckmäßig, wenn die pastenförmigen Komponenten A und B eine gleiche Oberflächenspannung besitzen. Diese Eigenschaft ist essentiell, damit eine homogene Vermischung der pastenförmigen Komponenten A und B erfolgen kann und Entmischungsprozesse vermieden werden. Die Oberflächenspannung kann durch Einsatz gleicher oder ähnlicher Monomere und durch Verwendung gleicher oder ähnlicher Polymere in den Komponenten A und B erreicht werden.

Als Methacrylatmonomere sind difunktionelle Methacrylate bevorzugt, besonders Ethylenglykoldimethacrylat, Butan-1,3-diol-dimethacrylat, Butan-1,4-diol-dimethacrylat und Hexan-1,6-diodimethacrylat. Daneben ist es auch möglich, andere di- und trifunktionelle Methacrylatmonomere zu verwenden. Ebenfalls können grundsätzlich auch monofunktionelle, destillierbare Methacrylate eingesetzt werden. Im Rahmen der Erfindung ist ebenfalls, zusätzliche Monomere mit Haftgruppen in den PMMA-Knochenzement zu integrieren, wie zum Beispiel Methacrylsäure-2-hydroxyethylester. Dadurch kann die Anbindung des PMMA-Knochenzementes an die Gelenkendoprothesen gezielt beeinflusst werden.

Als Poly-methylmethacrylat-copolymere sind Poly-methylmethacrylat-co-methylacrylat und Polymethylmethacrylat-co-styren bevorzugt. Daneben ist es auch möglich, andere Copolymere zu verwenden, die neben Methylmethacrylat auch aus anderen Alkylmethacrylaten aufgebaut sind.

Erwünscht ist es, wenn das Gewichtsverhältnis von Methylmethacrylatmonomere/Methylmethacrylatmonomeren zu löslichem Polymer zu im Methylmethacrylatmonomer/Methylmethacrylatmonomeren unlöslichem Polymer so ist, dass die pastösen Komponenten A und B klebfrei sind.

Bevorzugt wird ein Gewichtsverhältnis von 25-50 Gewichtsteilen Methacrylatmonomer/Methacrylatmonomeren zu 2-35 Gewichtsteilen lösliche Polymere und zu 40-70 Gewichtsteilen im Methylmethacrylatmonomer/Methylmethacrylatmonomeren unlösliches Polymer.

Als radikalischer Initiator sind Barbitursäurederivate bevorzugt, besonders Cyclohexylbarbitursäure. Unter dem Begriff Barbitursäurederivate werden Alkyl-, Cycloalkyl- und Arylderivate der Barbitursäure verstanden, wobei die Substituenten in den Positionen 1 und 5 oder nur 5 der Barbitursäure angeordnet sind. Unter diesen Begriff fallen auch Erdalkali- und Alkalisalze diese Barbitursäurederivate. Weiterhin sind auch Peroxide, wie z. B. Dibenzoylperoxid, als radikalische Initiatoren geeignet.

Als Akzelerator sind organische Kupfer(II)-salze bevorzugt, besonders Kuper(II)-2-ethylhexanoat, Kuper(II)-methacrylat, Kupfer(II)-hydroxid und basisches Kupfer(II)-carbonat. Daneben ist es aber auch möglich - bei Verwendung von Peroxiden als Initiator - N,N-Dimethylanilin, N,N-Dimethyl-p-toluidin und N,N-Bis(2-hydroxyethyl)-p-toluidin als Akzelerator einzusetzen.

Der radikalische Initiator und der Akzelerator sind zweckmäßig in solchen Konzentrationen enthalten, dass keine cytotoxischen Wirkungen auf Osteoblasten und Osteblasten-artige Zellen vom ausgehärteten Knochenzement ausgehen.

Im pastenförmigen PMMA-Knochenzement können Röntgenopaker enthalten sein, besonders Zirkoniumdioxid, Bariumsulfat, Tantal und biokompatible Calciumsalze.

Der erfindungsgemäße pastenförmige PMMA-Knochenzement kann auch pharmazeutische Wirkstoffe enthalten, wobei Antibiotika, Hormone, Wachstumsfaktoren und Antiphlogistika besonders bevorzugt sind. Als Antibiotika kommen vor allem Aminoglykosid-Antibiotika, Glykopeptid-Antibiotika, Fluorchinolon-Antibiotika, Lincosamid-Antibiotika und Oxazolidinon-Antibiotika in Betracht. Bevorzugt sind dabei Gentamicin, Tobramycin, Amikacin, Teicoplanin, Vancomycin, Ramoplanin, Dalbavancin, Moxifloxaxin, Ciprofloxacin, Lincosamin, Clindamycin und Linezolid bevorzugt. Die Antibiotika können in partikulärer oder auch in gelöster Form im erfindungsgemäßen PMMA-Knochenzement vorliegen.

Bevorzugt sind ein oder mehrere partikuläre oder im Methacrylatmonomer/Methacrylatmonomeren lösliche biokompatible Elastomere im pastenförmigen PMMA-Knochenzement enthalten, besonders bevorzugt Polybutadien.

Der erfindungsgemäße pastenförmige Knochenzement wird z. B. als selbsthärtender Kunststoff zur Fixierung von Totalendoprothesen und Revisionsendoprothesen verwendet und eignet sich besonders zur Zementierung von Hüft-, Knie- und Schultertotalendoprothesen.

Der erfindungsgemäße pastenförmige Knochenzement kann auch als selbsthärtendes Füllmaterial für die Vertebroplastie, Kyphoplastie und zur Femurhalsaugmentation Verwendung finden.

Der erfindungemäße pastenförmige PMMA-Knochenzement kann ferner als selbsthärtender Kunststoff zur Herstellung von lokalen Wirkstofffreisetzungssystemen verwendet werden. So ist es z. B. möglich, mit einem Antibiotikum enthaltenden erfindungsgemäßen PMMA-Knochenzement kugelförmige oder bohnenförmige Implantate zu formen, die als lokale Wirkstofffreisetzungssysteme eingesetzt werden können.

Die Erfindung wird durch nachstehende Beispiele erläutert, ohne jedoch die Erfindung einzuschränken. Teile- und Prozentangaben beziehen sich wie in der übrigen Beschreibung auf das Gewicht, sofern nicht anders angegeben.

### Beispiel 1

### Synthese des Calciumsalzes der 1-Cyclohexyl-5-ethyl-barbitursäure (CaCHEBA)

Es wurden in 50 ml Methanol 10,000 g (42 mmol) 1-Cyclohexyl-5-ethyl-barbitursäure und 1,621 g (21 mmol) Calciumhydroxid unter Rühren suspendiert. Anschließend wurde eine Stunde bei Raumtemperatur weiter gerührt. Danach wurde das Methanol mit einem Vakuumrotationsverdampfer abgezogen und der verbleibende Rückstand ohne weitere Reinigungsoperationen im Vakuum bis zur Massekonstanz getrocknet, wobei ein farbloser Feststoff anfiel.
Ausbeute: 11,000 g (97,8 %)
FT-IR ν (cm⁻¹): 3211; 3134; 3083; 2940; 2857; 1748; 1711; 1664; 1427; 1364; 1319; 1260; 1207; 1136; 1088; 1075; 1043; 998; 896; 858; 805; 768; 754; 736; 717; 666.

### Beispiel 2

### Herstellung eines Zirkoniumdioxid-Kupfercarbonat-Gemischs

Es wurden 20,000 g Zirkoniumdioxid-Pulver mit 40 mg basischem Kupfer(II)-carbonat (Cu-CO₃xCu(OH)₂) durch intensives Mahlen vermischt.

### Beispiel 3

### Herstellung eines Zirkoniumdioxid-Kupfercarbonat-Gemischs

Es wurden 10,000 g Zirkoniumdioxid-Pulver mit 20 mg Kupfer(II)-hydroxid (stabilisiertes Cu(OH)₂) durch intensives Mahlen vermischt.

### Beispiel 4

### Herstellung einer Polymer-Lösung 1

Es wurden bei Raumtemperatur unter intensivem Rühren in 85,0 g Hexan-1,6-diol-dimethacrylat 15,0 g eine Poly-methylmethacrylat-co-methylacrylat (Molmasse ca. 600000; ca. 50 % Methylacrylat) gelöst. Es entstand eine viskose klare Lösung.

### Beispiel 5

### Herstellung einer Polymer-Lösung 2

Es wurden bei Raumtemperatur unter intensivem Rühren in 80,0 g Hexan-1,6-diol-dimethacrylat 10,0 g eine Poly-methylmethacrylat-co-methylacrylat (Molmasse ca. 600000; ca. 50 % Methylacrylat) gelöst. Es entstand eine viskose klare Lösung.

Für die nachfolgend in den Beispielen 7-13 beschriebenen Pasten wurde ein partikuläres Polymethylmethacrylat-co-methylacrylat (Molmasse ca. 800000; ca. 5-8 % Methylacrylat, Korngröße < 63 µm) verwendet, das als Polymer 1 bezeichnet wird. Es wird weiterhin ein partikuläres, vernetztes Polymethylmethacrylat (Degacryl 6690) verwendet, das als Polymer 2 bezeichnet wird. Die Pasten A und B der Beispiele 6-13 wurden durch einfaches Verkneten der Komponenten hergestellt. Die Pasten A und B der Beispiele 6-13 waren klebfrei und konnten problemlos zu klebfreien Pasten 1-8 vermischt werden, die anschließend selbständig aushärteten.

### Beispiel 6

### Paste 1

Die Paste A und die Paste B stellten klebfreie streichbare, visuell homogene Pasten dar, die problemlos miteinander vermischt werden konnten.

| Pasten-Komponenten | Zusammensetzung | |
|---|---|---|
| | Paste A | Paste B |
| Polymer 1 | 4,998 g | 5,250 g |
| Polymer-Lösung 1 | 3,500 g | 3,500 g |
| Zirkoniumdioxid-Kupfercarbonat-Gemisch | 1,002 g | - |
| Zirkoniumdioxid | - | 1,000 g |
| CaCHEBA | 0,500 g | |
| 2-Ethyl-hexansäure | - | 0,200 g |
| ALIQUAT 336 | - | 0,050 g |

Nach Vermischung der Komponenten A und B war die entstandene Paste problemlos formbar und streichbar. Die Aushärtung setzte 2 Minuten und 50 Sekunden nach dem Vermischen ein.

### Beispiel 7

### Paste 2

| Pasten-Komponenten | Zusammensetzung | |
|---|---|---|
| | Paste A | Paste B |
| Polymer 1 | 4, 998 g | 5,250 g |
| Polymer-Lösung 1 | 3, 500 g | 3, 500 g |
| Zirkoniumdioxid-Kupfercarbonat-Gemisch | 0,501 g | - |
| Zirkoniumdioxid | 0,501 g | 1,000 g |
| CaCHEBA | 0,500 g | |
| 2-Ethyl-hexansäure | - | 0,200 g |
| ALIQUAT 336 | - | 0,050 g |

Die Aushärtung setzte 4 Minuten und 10 Sekunden nach dem Vermischen der Komponenten A und B ein.

### Beispiel 8

### Paste 3

| Pasten-Komponenten | Zusammensetzung | |
|---|---|---|
| | Paste A | Paste B |
| Polymer 1 | 4,998 g | 5,250 g |
| Polymer-Lösung 1 | 3,500 g | 3, 500 g |
| Zirkoniumdioxid-Kupfercarbonat-Gemisch | 0,250 g | - |
| Zirkoniumdioxid | 0,752 g | 1,000 g |
| CaCHEBA | 0,500 g | |
| 2-Ethyl-hexansäure | - | 0,200 g |
| ALIQUAT 336 | - | 0,050 g |

Die Aushärtung setzte 6 Minuten und 15 Sekunden nach dem Vermischen ein.

### Beispiel 9

### Paste 4

| Pasten-Komponenten | Zusammensetzung | |
|---|---|---|
| | Paste A | Paste B |
| Polymer 1 | 4,998 g | 5,250 g |
| Polymer-Lösung 1 | 3,500 g | 3,500 g |
| Zirkoniumdioxid-Kupfercarbonat-Gemisch | 1,002 g | - |
| Zirkoniumdioxid | - | 1,000 g |
| CaCHEBA | 0,500 g | |
| Octansäure | - | 0,200 g |
| ALIQUAT 336 | - | 0,050 g |

Nach Vermischung der Komponenten A und B war die Paste ebenfalls problemlos formbar und streichbar. Die Aushärtung setzte 3 Minuten und 5 Sekunden nach dem Vermischen ein.

### Beispiel 10

### Paste 5

| Pasten-Komponenten | Zusammensetzung | |
|---|---|---|
| | Paste A | Paste B |
| Polymer 1 | 4,998 g | 5,250 g |
| Polymer-Lösung 1 | 3,500 g | 3,500 g |
| Zirkoniumdioxid-Kupfercarbonat-Gemisch | 1,002 g | - |
| Zirkoniumdioxid | - | 1,000 g |
| CaCHEBA | 0,500 g | |
| Heptansäure | - | 0,200 g |
| ALIQUAT 336 | - | 0,050 g |

Nach Vermischung der Komponenten A und B war die Paste ebenfalls problemlos formbar und streichbar. Die Aushärtung setzte nach 3 Minuten und 5 Sekunden nach dem Vermischen ein.

### Beispiel 11

### Paste 6

| Pasten-Komponenten | Zusammensetzung | |
|---|---|---|
| | Paste A | Paste B |
| Polymer 1 | 4,998 g | 5,250 g |
| Polymer-Lösung 1 | 3,500 g | 3,500 g |
| Zirkoniumdioxid-Kupferhydroxid-Gemisch | 0,501 g | - |
| Zirkoniumdioxid | 0, 501g | 1,000 g |
| CaCHEBA | 0, 500 g | |
| Heptansäure | - | 0,200 g |
| ALIQUAT 336 | - | 0,050 g |

Nach Vermischung der Komponenten A und B war die Paste ebenfalls problemlos formbar und streichbar. Die Aushärtung setzte 3 Minuten und 20 Sekunden nach dem Vermischen ein.

### Beispiel 12

### Paste 7

| Pasten-Komponenten | Zusammensetzung | |
|---|---|---|
| | Paste A | Paste B |
| Polymer 1 | 4,998 g | 5,250 g |
| Polymer-Lösung 2 | 3, 500 g | 3,500 g |
| Zirkoniumdioxid-Kupferhydroxid-Gemisch | 0,501 g | - |
| Zirkoniumdioxid | 0,501 g | 1,000 g |
| CaCHEBA | 0,500 g | |
| 2-Ethyl-hexansäure | - | 0,200 g |
| ALIQUAT 336 | - | 0,050 g |

Nach Vermischung der Komponenten A und B war die Paste ebenfalls problemlos formbar und streichbar. Die Aushärtung begann 4 Minuten und 25 Sekunden nach dem Vermischen.

### Beispiel 13

### Paste 8

| Pasten-Komponenten | Zusammensetzung | |
|---|---|---|
| | Paste A | Paste B |
| Polymer 2 | 4,998 g | 5,250 g |
| Polymer-Lösung 1 | 3,500 g | 3, 500 g |
| Zirkoniumdioxid-Kupfercarbonat-Gemisch | 0,501 g | - |
| Zirkoniumdioxid | 0,501 g | 1,000 g |
| CaCHEBA | 0,500 g | |
| 2-Ethyl-hexansäure | - | 0,200 g |
| ALIQUAT 336 | - | 0,050 g |

Die Aushärtung setzte 4 Minuten und 5 Sekunden nach dem Vermischen der Komponenten A und B ein.

## Patentansprüche

1. Zweikomponentiger pastenförmiger PMMA-Knochenzement mit einer pastenförmige Komponente **A,** enthaltend
**AI.** mindestens ein destillierbares, radikalisch polymerisierbares Methacrylatmonomer,
**AII.** mindestens ein in **AI** lösliches Poly-methylmethacrylat-copolymer,
**AIII.** mindestens ein in **AI** nicht lösliches partikuläres vernetztes Polymethylmethacrylat oder vernetztes Poly-methylmethacrylat-co-methylacrylat mit einer Partikelgröße von höchstens 500 µm und
**AIV.** mindestens einen radikalischen Initiator und einer pastenförmigen Komponente **B**, enthaltend
**BI.** mindestens ein destillierbares, radikalisch polymerisierbares Methacrylatmonomer,
**BII.** mindestens ein in **BI** lösliches Poly-methylmethacrylat-copolymer,
**BIII.** mindestens ein in **BI** nicht lösliches partikuläres vernetztes Polymethylmethacrylat oder vernetztes Poly-methylmethacrylat-co-methylacrylat mit einer Partikelgröße kleiner 500 µm
**BIV.** und mindestens einen Akzelerator.

2. Zweikomponentiger pastenförmiger PMMA-Knochenzement nach Anspruch 1, **dadurch gekennzeichnet, dass** es sich bei den Methacrylatmonomeren um difunktionelle Methacrylate handelt.

3. Zweikomponentiger pastenförmiger PMMA-Knochenzement nach einem der vorstehenden Ansprüche, **dadurch gekennzeichnet, dass** es sich bei den Methacrylatmonomeren um solche der Gruppe bestehend aus Ethylenglykoldimethacrylat, Butan-1,3-diol-dimethacrylat, Butan-1,4-diol-dimethacrylat und Hexan-1,6-dio-dimethacrylat handelt.

4. Zweikomponentiger pastenförmiger PMMA-Knochenzement nach einem der vorstehenden Ansprüche, **dadurch gekennzeichnet, dass** die Polymere **AII** und **BII** Poly-methylmethacrylat-comethylacrylat oder Poly-methylmethacrylat-co-styren sind.

5. Zweikomponentiger pastenförmiger PMMA-Knochenzement nach einem der vorstehenden Ansprüche, **dadurch gekennzeichnet, dass** 25-50 Gewichtsteile (**AI+BI)** 2-35 Gewichtsteile **(AII+BII)** und 40-70 Gewichtsteile (**AIII+BIII)** vorhanden sind.

6. Zweikomponentiger pastenförmiger PMMA-Knochenzement nach einem der vorstehenden Ansprüche, **dadurch gekennzeichnet, dass** als radikalischer Initiator Barbitursäurederivate eingesetzt werden.

7. Zweikomponentiger pastenförmiger PMMA-Knochenzement nach einem der vorstehenden Ansprüche, **dadurch gekennzeichnet, dass** als radikalischer Initiator 1-Cyclohexyl-5-ethylbarbitursäure oder das Calciumsalz der 1-Cyclohexyl-5-ethyl-barbitursäure eingesetzt wird.

8. Zweikomponentiger pastenförmiger PMMA-Knochenzement nach einem der vorstehenden Ansprüche, **dadurch gekennzeichnet, dass** als Akzelerator organische Kupfer(II)-salze eingesetzt werden.

9. Zweikomponentiger pastenförmiger PMMA-Knochenzement nach einem der vorstehenden Ansprüche, **dadurch gekennzeichnet, dass** als Akzelerator Kupfer (II)-2-ethyl-hexanoat, Kupfer(II)-methacrylat, basisches Kupfer(II)carbonat oder Kupfer(II)-hydroxid eingesetzt wird.

10. Zweikomponentiger pastenförmiger PMMA-Knochenzement nach einem der vorstehenden Ansprüche, **dadurch gekennzeichnet, dass** der radikalische Initiator und der Akzelerator in solchen Konzentrationen enthalten sind, dass keine cytotoxischen Wirkungen auf Osteoblasten und Osteblasten-artige Zellen vom ausgehärteten Knochenzement ausgehen.

11. Zweikomponentiger pastenförmiger PMMA-Knochenzement nach einem der vorstehenden Ansprüche, **dadurch gekennzeichnet, dass** Röntgenopaker aus der Gruppe enthaltend Zirkoniumdioxid, Bariumsulfat, Tantal und biokompatible Calciumsalze enthalten sind.

12. Zweikomponentiger pastenförmiger PMMA-Knochenzement nach einem der vorstehenden Ansprüche, **dadurch gekennzeichnet, dass** pharmazeutische Wirkstoffe aus den Gruppen der Antibiotika, Hormone, Wachstumsfaktoren und Antiphlogistika enthalten sind.

13. Zweikomponentiger pastenförmiger PMMA-Knochenzement nach einem der vorstehenden Ansprüche, **dadurch gekennzeichnet, dass** ein oder mehrere biokompatible partikuläre oder im Methacrylatmonomer/Methacrylatmonomeren lösliche Elastomere enthalten sind.

14. Zweikomponentiger pastenförmiger PMMA-Knochenzement nach Anspruch 13, **dadurch gekennzeichnet, dass** Polybutadien als Elastomer enthalten ist.

15. Verwendung des zweikomponentigen pastenförmigen Knochenzementes nach mindestens einem der vorstehenden Ansprüche, zur Herstellung eines Mittels zur Fixierung von Totalendoprothesen und Revisionsendoprothesen.

16. Verwendung des zweikomponentigen pastenförmigen Knochenzementes nach mindestens einem der vorstehenden Ansprüche 1 bis 14 zur Herstellung eines Mittels für die Vertebroplastie, Kyphoplastie und Femurhalsaugmentation.

17. Zweikomponentiger pastenförmiger Knochenzement nach mindestens einem der vorstehenden Ansprüche 1 bis 14, zur Verwendung bei der Herstellung von lokalen Wirkstofffreisetzungs-systemen.

## Claims

1. Two-component pasty PMMA bone cement comprising a pasty component **A,** containing
**AI.** at least one methacrylate monomer for radical polymerisation that can be distilled;
**AII.** at least one poly-methylmethacrylate-co-polymer that is soluble in **AI;**
**AIII.** at least one particulate cross-linked poly-methylmethacrylate or cross-linked poly-methylmethacrylate-co-methylacrylate that is not soluble in **AI** and has a particle size of at most 500 µm; and
**AIV.** at least one radical initiator and a pasty component **B,** containing
**BI.** at least one methacrylate monomer for radical polymerisation that can be distilled;
**BII.** at least one poly-methylmethacrylate-co-polymer that is soluble in **BI**;
**BIII.** at least one particulate cross-linked poly-methylmethacrylate or cross-linked poly-methylmethacrylate-co-methylacrylate that is not soluble in **BI** and has a particle size of less than 500 µm;
**BIV.** and at least one accelerator.

2. Two-component pasty PMMA bone cement according to claim 1, **characterised in that** the methacrylate monomers are di-functional methacrylates.

3. Two-component pasty PMMA bone cement according to any one of the preceding claims, **characterised in that** the methacrylate monomers are from the group consisting of ethylene glycol dimethacrylate, butane-1,3-diol-dimethacrylate, butane-1,4-diol-dimethacrylate, and hexane-1,6-diol-dimethacrylate.

4. Two-component pasty PMMA bone cement according to any one of the preceding claims, **characterised in that** polymers **All** and **BII** are poly-methylmethacrylate-co-methylacrylate or poly-methylmethacrylate-co-styrene.

5. Two-component pasty PMMA bone cement according to any one of the preceding claims, **characterised in that** 25-50 parts by weight **(AI+BI),** 2-35 parts by weight **(AII+BII)** and 40-70 parts by weight **(AIII+BIII)** are present.

6. Two-component pasty PMMA bone cement according to any one of the preceding claims, **characterised in that** barbituric acid derivatives are used as radical initiator.

7. Two-component pasty PMMA bone cement according to any one of the preceding claims, **characterised in that** 1-cyclohexyl-5-ethyl-barbituric acid or the calcium salt of 1-cyclohexyl-5-ethyl-barbituric acid is used as radical initiator.

8. Two-component pasty PMMA bone cement according to any one of the preceding claims, **characterised in that** organic copper(II) salts are used as accelerator.

9. Two-component pasty PMMA bone cement according to any one of the preceding claims, **characterised in that** copper(II) 2-ethyl-hexanoate, copper(II) methacrylate, basic copper(II) carbonate or copper(II) hydroxide is used as accelerator.

10. Two-component pasty PMMA bone cement according to any one of the preceding claims, **characterised in that** it contains the radical initiator and the accelerator at appropriate concentrations such that the cured bone cement has no cytotoxic effects on osteoblasts and osteoblast-like cells.

11. Two-component pasty PMMA bone cement according to any one of the preceding claims, **characterised in that** it contains radiopaquers from the group containing zirconium dioxide, barium sulfate, tantalum, and biocompatible calcium salts.

12. Two-component pasty PMMA bone cement according to any one of the preceding claims, **characterised in that** it contains pharmaceutical agents from the groups of antibiotics, hormones, growth factors, and anti-phlogistic agents.

13. Two-component pasty PMMA bone cement according to any one of the preceding claims, **characterised in that** it contains one or more biocompatible elastomers that are particulate or soluble in the methacrylate monomer/methacrylate monomers.

14. Two-component pasty PMMA bone cement according to claim 13, **characterised in that** it contains polybutadiene as elastomer.

15. Use of the two-component pasty bone cement according to at least one of the preceding claims for production of a means for fixation of total endoprostheses and revision endoprostheses.

16. Use of the two-component pasty bone cement according to at least one of the preceding claims 1 to 14 for production of a means for vertebroplasty, kyphoplasty, and femoral neck augmentation.

17. Two-component pasty bone cement according to at least one of the preceding claims 1 to 14 for use in the production of local agent release systems.

## Revendications

1. Ciment osseux de PMMA pâteux à deux composantes avec une composante pâteuse **A** contenant
**AI**. au moins un monomère de méthacrylate polymérisable par voie radicalaire, pouvant être distillé,
**AII.** u moins un copolymère de poly-méthylméthacrylate soluble dans **AI,**
**AIII.** au moins un poly-méthylméthacrylate réticulé particulaire non soluble dans **AI** ou poly-méthylméthacrylate-co-méthylacrylate réticulé avec une taille particulaire de 500 µm maximum et
**AIV.** au moins un initiateur radicalaire
et une composante pâteuse **B** contenant
**BI.** au moins un monomère de méthacrylate polymérisable par voie radicalaire, pouvant être distillé,
**BII.** au moins un copolymère de poly-méthylméthacrylate soluble dans **BI**,
**BIII.** au moins un poly-méthylméthacrylate réticulé particulaire non soluble dans **BI** ou poly-méthylméthacrylate-co-méthylacrylate réticulé avec une taille particulaire inférieure à 500 µm
**BIV.** et au moins un accélérateur.

2. Ciment osseux de PMMA pâteux à deux composantes selon la revendication 1, **caractérisé en ce qu'**il s'agit pour les monomères de méthacrylate de méthacrylates difonctionnels.

3. Ciment osseux de PMMA pâteux à deux composantes selon une des revendications précédentes, **caractérisé en ce qu'**il s'agit pour les monomères de méthacrylate de ceux du groupe constitué du diméthacrylate d'éthylèneglycol, butane-1,3-diol-diméthacrylate, butane-1,4-diol-diméthacrylate et hexane-1,6-diol-diméthacrylate.

4. Ciment osseux de PMMA pâteux à deux composantes selon une des revendications précédentes, **caractérisé en ce que** les polymères **AII** et **BII** sont un poly-méthylméthacrylate-co-méthylacrylate ou poly-méthylméthacrylate-co-styrène.

5. Ciment osseux de PMMA pâteux à deux composantes selon une des revendications précédentes, **caractérisé en ce que** 25 à 50 parties en poids **(AI+BI),** 2 à 35 parties en poids **(AII+BII)** et 40 à 70 parties en poids **(AIII+BIII)** sont présentes.

6. Ciment osseux de PMMA pâteux à deux composantes selon une des revendications précédentes, **caractérisé en ce que** des dérivés d'acide barbiturique sont utilisés en tant qu'initiateur radicalaire.

7. Ciment osseux de PMMA pâteux à deux composantes selon une des revendications précédentes, **caractérisé en ce que** de l'acide 1-cyclohexyl-5-éthyl-barbiturique ou le sel de calcium de l'acide 1-cyclohexyl-5-éthyl-barbiturique est utilisé en tant qu'initiateur radicalaire.

8. Ciment osseux de PMMA pâteux à deux composantes selon une des revendications précédentes, **caractérisé en ce que** des sels de cuivre(II) organiques sont utilisés en tant qu'accélérateur.

9. Ciment osseux de PMMA pâteux à deux composantes selon une des revendications précédentes, **caractérisé en ce que** du 2-éthyl-hexanoate de cuivre(II), du méthacrylate de cuivre(II), du carbonate de cuivre(II) basique ou de l'hydroxyde de cuivre(II) est utilisé en tant qu'accélérateur.

10. Ciment osseux de PMMA pâteux à deux composantes selon une des revendications précédentes, **caractérisé en ce que** l'initiateur radicalaire et l'accélérateur sont contenus en des concentrations telles qu'aucune action cytotoxique sur des ostéoblastes et cellules de type ostéoblastes ne provient du ciment osseux durci.

11. Ciment osseux de PMMA pâteux à deux composantes selon une des revendications précédentes, **caractérisé en ce que** des opacifiants radiographiques provenant du groupe contenant le dioxyde de zirconium, sulfate de baryum, tantale et des sels de calcium biocompatibles sont contenus.

12. Ciment osseux de PMMA pâteux à deux composantes selon une des revendications précédentes, **caractérisé en ce que** des ingrédients actifs pharmaceutiques provenant des groupes des antibiotiques, hormones, facteurs de croissance et anti-inflammatoires sont contenus.

13. Ciment osseux de PMMA pâteux à deux composantes selon une des revendications précédentes, **caractérisé en ce qu'**un ou plusieurs élastomères particulaires biocompatibles ou solubles dans le monomère de méthacrylate/monomères de méthacrylate sont contenus.

14. Ciment osseux de PMMA pâteux à deux composantes selon la revendication 13, **caractérisé en ce que** du polybutadiène est contenu en tant qu'élastomère.

15. Utilisation du ciment osseux pâteux à deux composantes selon au moins une des revendications précédentes pour la fabrication d'un moyen pour la fixation d'endoprothèses totales et d'endoprothèses de révision.

16. Utilisation du ciment osseux pâteux à deux composantes selon au moins une des revendications précédentes 1 à 14 pour la fabrication d'un moyen pour la vertébroplastie, la kyphoplastie et l'augmentation du col du fémur.

17. Ciment osseux pâteux à deux composantes selon au moins une des revendications précédentes 1 à 14 pour l'utilisation lors de la fabrication de systèmes de libération d'ingrédient actif locaux.
